# DEMANDE DE BREVET EUROPEEN

(11) **EP 1 764 054 A1**
(43) Date de publication de la demande: **21.03.2007**
(21) Numéro de dépôt: 06350010.2
(22) Date de dépôt: 31.08.2006
(51) Int. Cl.: A61B 17/80

(54) **Dispositif d'ostéosynthèse**

(30) Priorité: 19.09.2005 FR 0509523
(71) Demandeur: D.L.P., 44800 Saint Herblain (FR)
(72) Inventeur: Derouet, Guillaume, 44800 Saint Herblain (FR)
(74) Mandataire: Tournel, Jean Louis

(57) **Abrégé**

L'invention a pour objet un dispositif d'ostéosynthèse comprenant une plaque (2) pourvue de passage de vis et des vis (3) caractérisé en ce qu'au moins un des passages (4) de vis est conformé pour former un outil (5) venant former une rainure hélicoïdale sur la partie (6) proximale d'une vis (3).

## Description

L'invention se rapporte à un dispositif d'ostéosynthèse.

Elle se rapporte plus particulièrement mais non limitativement aux dispositifs d'ostéosynthèse utilisés pour la micro-chirurgie.

Lors d'une fracture, il est souvent nécessaire de maintenir en place les os au moyen d'une plaque fixée au long de l'os fracturé, le temps nécessaire à la formation de l'os et donc à la consolidation de la fracture.

La plaque est bien évidemment adaptée à la morphologie de l'os qu'elle doit fixer.

Des vis engagées dans des passages de vis présentés par la plaque viennent fixer la plaque d'ostéosynthèse sur les fragments d'os.

Cette plaque doit empêcher les mouvements d'un fragment osseux par rapport à un autre car cela empêcherait la consolidation correcte de la fracture..

On recherche de ce fait un blocage de la vis sur la plaque.

Pour un blocage de la vis sur la plaque, celle-ci comprend, en plus du filet de vis classique qui va pénétrer dans l'os, un filet de vis supplémentaire pour s'engager, avec celui bordant le passage de vis.

La vis doit alors s'engager selon une direction prédéfinie lors de la construction de la plaque d'ostéosynthèse. Cette direction est définie par l'orientation du filet de vis présenté par la plaque ou alors il faut faire appel à des systèmes plus compliqués et plus encombrants.

Suivant l'endroit de la fracture, les caractéristiques de cette plaque peuvent être très différentes.

En effet, une plaque posée sur une phalange ne subit pas le même effort qu'une plaque posée sur un radius.

Pour ces os relativement petits, il est nécessaire que la plaque soit relativement mince.

Cela est rendu possible car les efforts qu'elle va subir sont beaucoup plus faibles.

Il est important également de pouvoir orienter les vis selon différentes directions.

Il s'agit là d'une difficulté importante pour les plaques de faible épaisseur.

Les dispositions connues à ce jour pour les plaques plus épaisses ne conviennent pas.

Cependant, il est connu un dispositif d'ostéosynthèse (WO-A-2004/032751) qui comprend une plaque et des vis.

Cette plaque présente au niveau des passages de vis, un insert en matériau tendre en forme de bague pouvant être taraudé par une vis comprenant en son extrémité située au plus près de la tête de la dite vis, un filet taraudeur à même de créer dans ce matériau tendre une rainure hélicoïdale complémentaire.

La vis peut donc être engagée dans le passage de vis selon une orientation angulaire non fixée par avance et cette vis va former, dans le matériau plastique une gorge hélicoïdale.

Ceci permettra de bloquer la vis dans la bague en plastique.

Cette solution semble intéressante mais si par mégarde une vis est engagée selon une mauvaise direction, la bague, alors taraudée, ne peut plus recevoir une vis selon une deuxième direction.

Il faut alors procéder à un échange de plaque ce qui n'est pas satisfaisant.

Par ailleurs en rapportant cet insert sur la plaque, on crée nécessairement une surépaisseur pour le calage de l'insert.

L'invention se propose d'apporter une solution.

A cet effet, l'invention a pour objet un dispositif d'ostéosynthèse comprenant une plaque avec des passages de vis, ce dispositif étant caractérisé en ce que le pourtour d'au moins un des passages de vis est conformé pour former un outil venant former une rainure hélicoïdale sur la partie proximale d'une vis.

L'invention sera bien comprise à l'aide de la description ci-après faite à titre d'exemple non limitatif en regard du dessin qui représente :

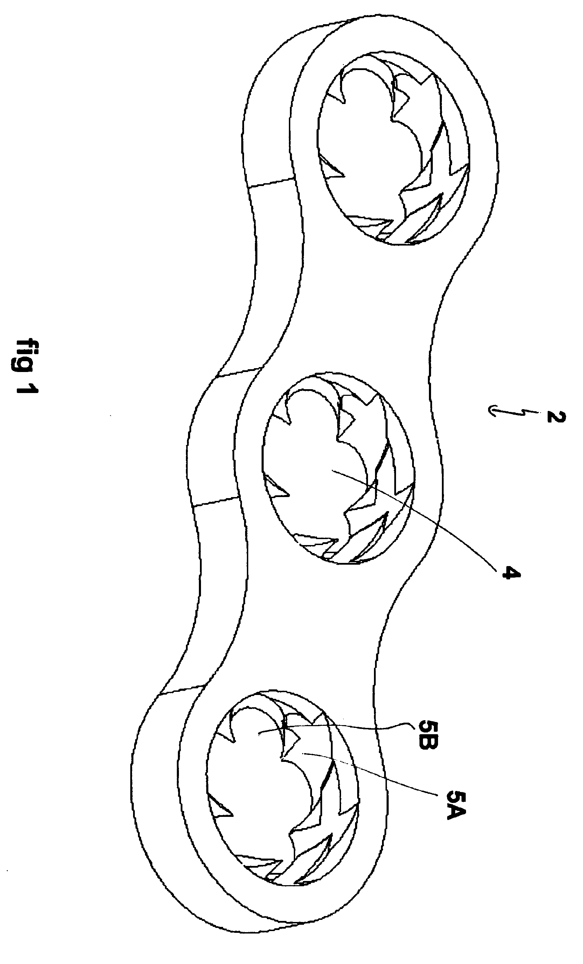

FIG 1 : Une plaque d'ostéosynthèse

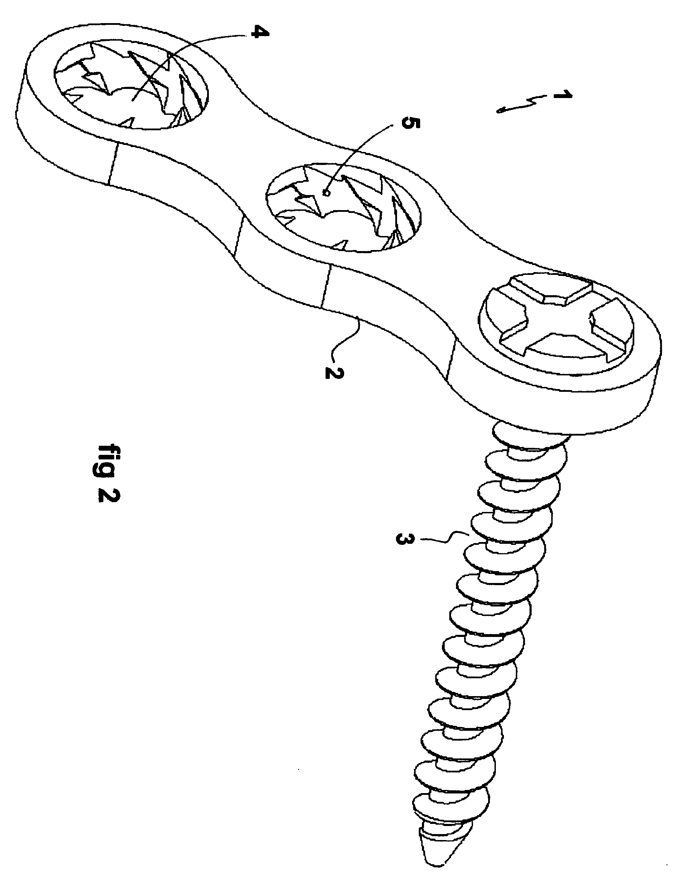

FIG 2 : Une plaque d'ostéosynthèse et sa vis
FIG 3 : Une vis

En se reportant au dessin, on voit un dispositif 1 d'ostéosynthèse destiné, par exemple, à maintenir deux fragments d'os. Ce dispositif comprend une plaque 2 conformée selon l'utilisation qui en sera faite et des vis 3.

Il s'agit principalement de traiter les petits os de la main ou du pied. Cette plaque est donc de faible épaisseur.

Cette plaque comporte des passages 4 de vis.

Selon l'invention, au moins un des passages 4 de vis est conformé pour former un outil 5 venant former une rainure hélicoïdale sur la partie 6 proximale d'une vis 3.

Par partie proximale, on comprendra la partie située au niveau de la tête de vis.

La gorge hélicoïdale sera formée sur toute ou partie de cette tête.

Cet outil 5 encore appelé filière va, soit enlever de la matière pour former une gorge hélicoïdale sur la partie 3 proximale de la vis, soit déformer cette matière.

Au moins, la zone de la tête de vis qui sera filetée sera de dureté plus faible que la matière de l'outil 5 formé ou rapporté sur la plaque 2.

Avant son utilisation, la vis se présente sous la forme d'un corps cylindrique dont la partie distale 7 présente un filet de vis qui va s'engager dans l'os. La partie 6 proximale est, avant utilisation, lisse et donc dépourvue de rainure hélicoïdale.

Dans un forme de réalisation, la partie de la vis devant être attaquée par l'outil 5 est surmoulée sur le corps de la vis. Par exemple, la partie proximale de la vis est en polyétheréthercétone (PEEK).

Dans cette zone 6, dite de surmoulage, le corps de la vis est de section réduite.

Le surmoulage sera donc calé axialement. Des formes permettent de caler en rotation le surmoulage.

Avec cette solution, même si la vis est engagée selon un axe qui ne convient pas, il suffit de remplacer la vis par une vis non utilisée.

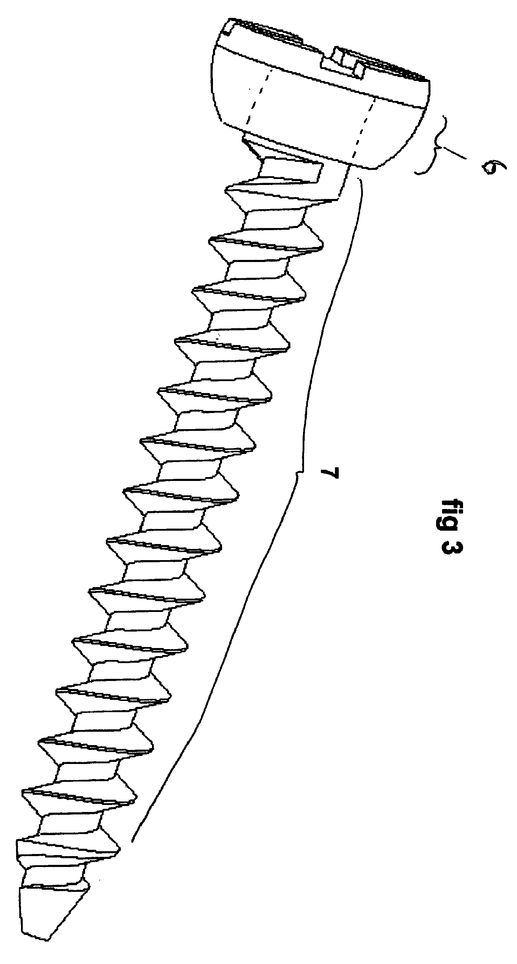

La figure 3 montre la vis non utilisée et elle ne présente pas de rainure hélicoidale.

Comme on peut le voir, la partie 6 proximale de la vis se présente sous la forme approximative d'un hémisphère situé sous l'empreinte 7 destinée à la manoeuvre de la vis. L'outil 5 comprend des dents 5A réparties sur la circonférence du passage de vis. Ces dents font saillies par rapport à une face interne sensiblement hémisphérique. Elles sont taillées et implantées pour former une hélice d'un pas donné et d'un profil donné.

Entre deux dents 5A, une échancrure 5B est donc prévue pour l'évacuation du copeau formé lors de la création de la gorge hélicoïdale.

Ainsi, il est possible d'orienter la vis selon des directions autres que la normale au plan du passage de vis.

Le pas formé par l'outil 5 peut être différent de celui de la vis qui s'engage dans l'os en sorte de pouvoir plaquer la plaque d'ostéosynthèse sur l'os.

La plaque et/ou les vis sont en matériau biocompatible tel l'acier inoxydable ou le titane.

## Revendications

1. Dispositif d'ostéosynthèse comprenant une plaque (2) pourvue de passages de vis **caractérisé en ce qu'**au moins un des passages (4) de vis est conformé pour former un outil (5) venant former une rainure hélicoïdale sur la partie (6) proximale d'une vis (3).

2. Dispositif d'ostéosynthèse selon la revendication 1 **caractérisé en ce que** l'outil (5) est formé ou rapporté sur la plaque d'ostéosynthèse.

3. Dispositif d'ostéosynthèse selon la revendication 1 **caractérisé en ce qu'**au moins la partie proximale de la tête de vis destinée à être engagée dans le passage de vis est en matériau moins dur que celui de l'outil.

4. Dispositif d'ostéosynthèse selon la revendication 3 **caractérisé en ce que** la partie (6) proximale de la tête de vis est surmoulée sur le corps de la vis.

5. Dispositif d'ostéosynthèse selon la revendication 4 **caractérisé en ce que** la partie (6) proximale de la vis est en polyétheréthercétone (PEEK).

6. Dispositif d'ostéosynthèse selon l'une quelconque des revendications précédentes **caractérisé en ce que** :
- la partie (6) proximale de la vis se présente sous la forme approximative d'un hémisphère situé sous l'empreinte (7) destinée à la manoeuvre de la vis et,
- l'outil (5) comprend des dents (5A) réparties sur la circonférence du passage de vis, ces dents faisant saillies par rapport à une face interne sensiblement hémisphérique.

7. Dispositif d'ostéosynthèse selon la revendication 6 **caractérisé en ce qu'**entre les dents est prévue une échancrure pour le copeau formé.
